# EUROPEAN PATENT APPLICATION

(11) **EP 4 717 768 A1**
(43) Date of publication of application: **01.04.2026**
(21) Application number: 23938203.9
(22) Date of filing: 27.10.2023
(51) Int. Cl.: C12N 15/00, C12N 5/00, A01H 5/00

(54) **PLANT PLASMODIOPHOROMYCETE RESPONSIVE PROMOTER AND USE THEREOF**

(30) Priority: 22.05.2023 CN 202310577587
(71) Applicant: INSTITUTE OF GENETICS AND DEVELOPMENTAL BIOLOGY, CHINESE ACADEMY OF SCIENCES, Chaoyang District Beijing 100101 (CN)
(72) Inventor: ZHOU, Jianmin, Beijing 100101 (CN); WANG, Wei, Beijing 100101 (CN); ZHANG, Wenjing, Beijing 100101 (CN); DONG, Xiaojing, Beijing 100101 (CN)
(74) Representative: BASF IP Association
(86) International application number: PCT/CN2023/127233
(87) International publication number: WO 2024/239535

(57) **Abstract**

The present invention discloses a plant Plasmodiophora-responsive promoter and the use thereof. The plant Plasmodiophora-responsive promoter is A1) a DNA molecule as shown in sequence 3, or A2) a DNA molecule having 50% or more identity to the nucleotide sequence as defined in A1) and having promoter function. It is proved by experiments that the promoter disclosed in the present invention comprises important elements responsive to *Plasmodiophora* infection, and is a *Plasmodiophora-*responsive promoter. The promoter is very important for mediating resistance to *Plasmodiophora,* and has an important application value.

## Description

### Technical Field

The present invention belongs to the field of biotechnology, and in particular relates to a plant Plasmodiophora-responsive promoter and the use thereof.

### Background Art

Cruciferous plants such as *Brassica napus* are important sources of oil. However, in recent years, diseases of cruciferous oil crops caused by *Plasmodiophora* have severely affected their yield and quality. In previous studies, a large number of plant resistance (*R*) genes have been identified, most of which belong to NLR genes. NLR genes contain a nucleotide-binding site and a leucine-rich repeat sequence, and play an important role in plant immunity, especially in the ETI response. In addition, another class of *R* genes has also been identified in rice and pepper. The proteins encoded by these genes can directly kill plant cells infected by pathogens, thereby achieving resistance to the pathogens. This class of genes is known as Executor (*E*) *R* genes. Although both NLR and Executor R proteins can activate plant immunity, their activation mechanisms are quite different. NLR proteins are typically in an auto-inhibited state and only switch to an activated state to function after recognizing effector proteins from pathogens. Executor *R* genes regulate their activity at the transcriptional level. Under normal conditions, Executor *R* genes remain silent; upon pathogen invasion, their effector proteins bind to the promoters of the Executor *R* genes, inducing the expression of the genes and triggering disease resistance in plants.

A promoter is a DNA sequence located in a region upstream of the 5'end of a structural gene. It can accurately bind to transcription-related proteins such as RNA polymerase, possesses transcription initiation specificity, and is an important component for gene expression regulation, controlling the initiation timing and expression level of gene expression. Plant gene promoters contain a variety of important cis-acting elements, which participate in regulating the expression of corresponding downstream genes at the transcriptional level, conferring spatiotemporal expression specificity to the genes. Therefore, the isolation and functional analysis of promoters are important contents in plant genetic engineering research.

### The Disclosure of the Invention

An object of the present invention is to provide a plant *Plasmodiophora*-responsive promoter and the use thereof.

In a first aspect, the present invention sets forth a DNA molecule, wherein the DNA molecule is A1) or A2) as follows:
A1) a DNA molecule as shown in sequence 3;
A2) a DNA molecule having 50% or more identity to the nucleotide sequence as defined in A1) and having promoter function.

Those of ordinary skill in the art can readily use known methods, such as directed evolution and site-directed mutagenesis, to mutate the nucleotide sequence of the DNA molecule of the present invention. Those nucleotides that are artificially modified and have 50% or more identity to the nucleotide sequence of the DNA molecule provided by the present invention, provided that they have promoter function, are derived from the nucleotide sequence of the present invention and are equivalent to the sequence of the present invention.

The term "identity" as used herein refers to sequence similarity to a natural nucleic acid sequence. "Identity" includes a nucleotide sequence having 50% or more, or 55% or more, or 60% or more, or 65% or more, or 70% or more, or 75% or more, or 80% or more, or 85% or more, or 90% or more, or 95% or more identity to the nucleotide sequence of the DNA molecule of the present invention. Identity can be evaluated by visual inspection or with computer software. Using computer software, the identity between two or more sequences can be expressed as a percentage (%), which can be used to evaluate the identity between related sequences.

In a second aspect, the present invention sets forth a biological material related to the aforementioned DNA molecule, wherein the biological material is any one of B1) to B4) as follows:
B1) an expression cassette containing the aforementioned DNA molecule;
B2) a recombinant vector containing the aforementioned DNA molecule, or a recombinant vector containing the expression cassette of B1);
B3) a recombinant microorganism containing the aforementioned DNA molecule, a recombinant microorganism containing the expression cassette of B1), or a recombinant microorganism containing the recombinant vector of B2);
B4) a transgenic plant cell line containing the aforementioned DNA molecule, or a transgenic plant cell line containing the expression cassette of B1).

In the aforementioned biological material, the expression cassette (5' to 3') may comprise a promoter region (consisting of the DNA molecule), a transcription initiation region, a gene-of-interest region, a transcription termination region, and optionally a translation termination region. The promoter region and the gene-of-interest region may be native/similar to the host cell, or the promoter region and the gene-of-interest region may be native/similar to each other, or the promoter region and/or the gene-of-interest region are heterologous to the host or to each other. The term "heterologous" means that the sequence is a sequence derived from a foreign species, or, if from the same species, is substantially modified in composition and/or genomic locus from the native form by deliberate human intervention. The transcription termination region, optionally contained, may be homologous to the transcription initiation region, homologous to the operably linked gene-of-interest region, or homologous to the host; or the gene-of-interest region or the host are foreign or heterologous.

The expression cassette may further comprise a 5' leader sequence. The 5' leader sequence can enhance translation.

During preparation of the expression cassette, adapters or linkers may be applied to link DNA fragments, or other manipulations may be involved to provide appropriate restriction sites, remove excess DNA, remove restriction sites, etc. For this purpose, in vitro mutagenesis, primer repair, restriction enzyme digestion, annealing, and replacement, such as transitions and transversions, may be performed.

The expression cassette may further comprise a selective marker gene for screening transformed cells. The selective marker gene can be used to screen transformed cells or tissues. Marker genes include genes encoding antibiotic resistance. Other selective markers include phenotypic markers such as fluorescent proteins. The selective markers listed above are non-limiting. Any selective marker gene can be used in the present invention.

In the aforementioned biological material, the vector may be a plasmid, cosmid, phage, or viral vector.

In a particular embodiment of the present invention, the recombinant vector is *pRCR1*^{Est}*::GUS*. The *pRCR1*^{Est}*::GUS* is a vector obtained by ligating the DNA molecule as shown in sequence 3 into the Hindlll and Xbal restriction sites of the pCAMBIA1300-GUS vector.

In another particular embodiment of the present invention, the recombinant vector is *pRCR1*^{Est}*::nls-3***mVenus.* The *pRCR1*^{Est}*::nls-3***mVenus* is a vector obtained by ligating the DNA molecule as shown in sequence 3 into the Hindlll and EcoRI restriction sites of the pGREEN-nls-3*mVenus vector.

In a further particular embodiment of the present invention, the recombinant vector is *pRCR1*^{Est}*::RCR*^{L*er*}. The *pRCR1*^{Est}*::RCR*^{L*er*} is a vector obtained by ligating the DNA molecule as shown in sequence 5 into the Sall and Xball restriction sites of the pGREEN vector.

In the aforementioned biological material, the microorganism may be a yeast, a bacterium, an alga, or a fungus. The bacterium may be an *Agrobacterium* (e.g., *Agrobacterium* GV3101).

In the aforementioned biological material, the transgenic plant cell line does not include a propagating material. The transgenic plant is understood to include not only the first-generation transgenic plant obtained by transforming the DNA molecule into a recipient plant, but also a progeny thereof. For the transgenic plant, the DNA molecule can be propagated within the species, or can also be transferred into other varieties of the same species, especially including commercial varieties, using conventional breeding techniques. The transgenic plant includes seeds, callus, whole plants, and cells.

In a third aspect, the present invention sets forth the new use of the aforementioned DNA molecule or biological material.

The present invention sets forth the use of the aforementioned DNA molecule as a promoter or a Plasmodiophora-responsive promoter.

The present invention sets forth the use of the aforementioned DNA molecule or biological material in initiating the expression of a gene of interest or in increasing the expression level of a gene of interest.

The present invention sets forth the use of the aforementioned DNA molecule or biological material in the preparation of a transgenic plant or in plant breeding.

In any one of the aforementioned uses, the purpose of preparing a transgenic plant or plant breeding is to breed a disease-resistant plant variety (e.g., a clubroot-resistant plant variety).

In a fourth aspect, the present invention sets forth a method for expressing a gene of interest or a method for increasing the expression level of a gene of interest, wherein the method is any one of C1)-C4) as follows:
C1) comprising the step of using the aforementioned DNA molecule as a promoter or a Plasmodiophora-responsive promoter to initiate the expression of the gene of interest;
C2) comprising the step of inserting the aforementioned DNA molecule upstream of the gene of interest or an enhancer to initiate the expression of the gene of interest;
C3) comprising the step of inserting the gene of interest downstream of the aforementioned DNA molecule in the aforementioned expression cassette to initiate the expression of the gene of interest by the DNA molecule;
C4) comprising the step of inserting the gene of interest downstream of the aforementioned DNA molecule in the aforementioned recombinant vector to initiate the expression of the gene of interest by the DNA molecule.

The use of any one of the aforementioned methods in the preparation or breeding of a transgenic plant also falls within the scope of protection of the present invention.

The expression of any one of the aforementioned genes of interest can be initiating expression of the gene of interest in a plant, can be initiating expression of the gene of interest under *Plasmodiophora* infection conditions, or can be increasing the expression level of the gene of interest under *Plasmodiophora* infection conditions.

Any one of the aforementioned genes of interest may be an endogenous plant gene (e.g.,*RCR1*^{Est} gene or *RCR1*^{L*er*} gene) or a foreign gene (e.g., GUS gene or *mVENUS* gene). The nucleotide sequence of the *RCR1*^{Est} gene is as shown in sequence 1. The nucleotide sequence of the *RCR1*^{L*er*} gene is as shown in sequence 2.

Any one of the aforementioned *Plasmodiophora* may be *Plasmodiophora* physiological race No. 4.

The method for infection with any of the aforementioned *Plasmodiophora* can be irrigating plant roots with a *Plasmodiophora* suspension. In a particular embodiment of the present invention, the concentration of the *Plasmodiophora* suspension may be 1.0×10⁷ spores/mL.

Any one of the aforementioned plants may be a dicotyledonous plant or a monocotyledonous plant.

Further, the dicotyledonous plant may be a cruciferous plant.

Still further, the cruciferous plant may be *Arabidopsis thaliana* or *Brassica napus.*

In a particular embodiment of the present invention, the *Arabidopsis thaliana* is *Arabidopsis thaliana* ecotype Col-0 or Ler or Est-1.

### Brief Description of the Drawings

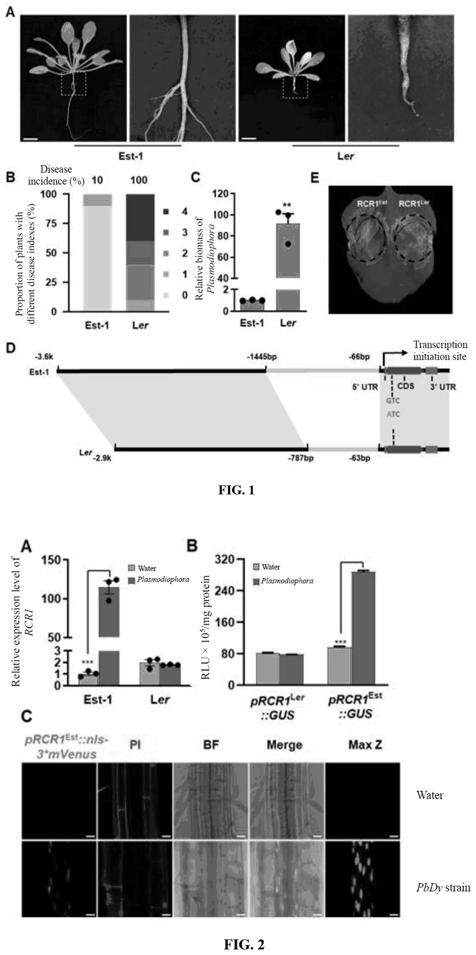

FIG.1 shows analysis of resistance to *Plasmodiophora* in clubroot-susceptible material Ler and clubroot-resistant material Est-1, and comparative analysis of *Plasmodiophora* resistance gene RCR1 haplotypes, where panel A shows disease symptoms of clubroot-susceptible material L*er* and clubroot-resistant material Est-1 21 days after inoculation with *Plasmodiophora,* panel B shows disease incidence and disease index of clubroot-susceptible material Ler and clubroot-resistant material Est-1 21 days after inoculation with *Plasmodiophora,* panel C shows the biomass of *Plasmodiophora* in clubroot-susceptible material Ler and clubroot-resistant material Est-1 21 days after inoculation with *Plasmodiophora* (n ≥ 12), panel D shows a comparison of RCR1 haplotypes in clubroot-susceptible material Ler and clubroot-resistant material Est-1 (red arrows indicate coding regions; blue rectangles indicate UTR regions; pink and green lines represent divergent regions in *RCR1*^{Est} and *RCR1*^{L*er*} promoters), and panel E shows that transient expression of either *RCR1*^{L*er*} or *RCR1*^{Est} can induce cell death in tobacco.
FIG. 2 shows that the *RCR1*^{Est} promoter contains important elements responsive to *Plasmodiophora* infection, where panel A shows the expression level of *RCR1* in the underground parts of clubroot-susceptible material Ler and clubroot-resistant material Est-1 11 days after inoculation with *Plasmodiophora,* panel B shows the GUS activity in the underground parts of transgenic plants *pRCR1*^{L}*^{er}::GUS* and *pRCR1*^{Est}*::GUS* determined using the 4-MUG fluorometric assay 11 days after inoculation with *Plasmodiophora,* and panel C shows the fluorescent protein expression in the roots of transgenic plant *pRCR1*^{Est}*::nls-3***mVenus* 11 days after inoculation with *Plasmodiophora* (*pRCR1*^{L}*^{er}::GUS* and *pRCR1*^{Est}*::GUS* represent transgenic plants in the Col-0 background in which GUS expression is driven by the *RCR1*^{L*er*} or *RCR1*^{Est} promoter, respectively, and *pRCR1*^{Est}*::nls-3*mVenus* represents a transgenic plant in the Col-0 background in which *NLS-3*mVenus* expression is driven by the *RCR1*^{Est} promoter).

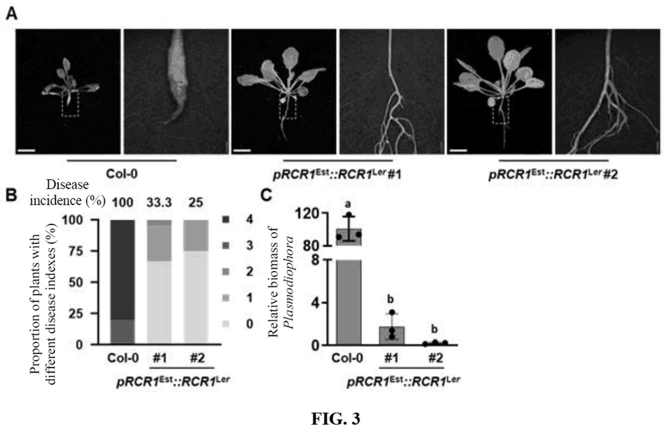

FIG. 3 shows that the *RCR1*^{Est} promoter is essential for RCR1-mediated resistance to *Plasmodiophora,* where panel A shows disease symptoms of Col-0 and transgenic plant *pRCR1*^{Est}*::RCR1*^{L*er*} 21 days after inoculation with *Plasmodiophora,* panel B shows disease incidence and disease index of Col-0 and transgenic plant *pRCR1*^{Est}*::RCR1*^{L*er*} 21 days after inoculation with *Plasmodiophora,* and panel C shows the relative biomass of *Plasmodiophora* in Col-0 and transgenic plant *pRCR1*^{Est}*::RCR1*^{L*er*} 21 days after inoculation with *Plasmodiophora* (n ≥ 12).

### Best mode for implementing the present invention

The following examples facilitate a better understanding of the present invention, but do not limit the present invention. The test methods in the following examples are all conventional methods, unless otherwise specified. The test materials used in the following examples, unless otherwise specified, are purchased from conventional biochemical reagent suppliers. In the following examples, quantitative tests all set up three replicate experiments, and the results are averaged.

The pER8 vector in the following examples is described in the document "Zuo, J. et al. Technical advance: an estrogen receptor-based transactivator XVE mediates highly inducible gene expression in transgenic plants. Plant J. 24, 265-273 (2000).", publicly available from the Institute of Genetics and Developmental Biology, Chinese Academy of Sciences. This biological material is to be used solely for repeating the experiments related to the present invention and shall not be used for any other purpose.

The pGREEN-nls-3*mVenus vector in the following examples is described in the document "Zhou, F. et al. Co-incidence of damage and microbial patterns controls localized immune responses in roots. Cell 180, 440-453(2020).", publicly available from the Institute of Genetics and Developmental Biology, Chinese Academy of Sciences. This biological material is to be used solely for repeating the experiments related to the present invention and shall not be used for any other purpose.

The pCAMBIA1300-GUS vector in the following examples is described in the document "Wang, Y. et al. Vascular-specific expression of Gastrodia antifungal protein gene significantly enhanced cotton Verticillium wilt resistance. Plant Biotechnol J. 181498-1500 (2020).", publicly available from the Institute of Genetics and Developmental Biology, Chinese Academy of Sciences. This biological material is to be used solely for repeating the experiments related to the present invention and shall not be used for any other purpose.

The *Agrobacterium* GV3101 in the following examples is described in the document "Liang, XX. et al. Ligand-triggered de-repression of Arabidopsis heterotrimeric G proteins coupled to immune receptor kinases. Cell Research 28, 529-543 (2019).", publicly available from the Institute of Genetics and Developmental Biology, Chinese Academy of Sciences. This biological material is to be used solely for repeating the experiments related to the present invention and shall not be used for any other purpose.

### Example 1. Analysis of resistance to Plasmodiophora in Arabidopsis thaliana ecotypes Est-1 and Ler

### 1. Inoculation with Plasmodiophora

*Arabidopsis thaliana* ecotype Est-1 (clubroot-resistant material) and *Arabidopsis thaliana* ecotype Ler (clubroot-susceptible material) were used as experimental materials for inoculation experiments with *Plasmodiophora,* respectively. Before inoculation, all experimental materials were cultured under conventional conditions (10 hours of light, 14 hours of darkness, at a temperature of 22°C). When the plants had grown for about 14 days, the root infection experiment with *Plasmodiophora* was performed. The specific steps are as follows:
1) The root pieces of the collected plants containing *Plasmodiophora* (identified as physiological race No. 4) from the Dayi region of Sichuan province were cut into small pieces using a blade, water was added, and then the mixture was broken in a tissue crusher to obtain a mixture containing *Plasmodiophora* suspension and broken tissue.
2) The mixture containing *Plasmodiophora* suspension and broken tissue obtained in step 1) was filtered using 8 layers of gauze to obtain a relatively clean *Plasmodiophora* suspension.
3) The spore concentration of *Plasmodiophora* in the *Plasmodiophora* suspension obtained in step 2) was measured using a hemocytometer, and the concentration of the *Plasmodiophora* suspension was diluted to 1.0×10⁷ spores/mL.
*4) Arabidopsis thaliana* was inoculated using the root irrigation method, that is, 1 mL of the diluted *Plasmodiophora* suspension obtained in step 3) was drawn using a pipette, and used to irrigate the roots of the *Arabidopsis thaliana.*

### 2. Statistics of disease index and disease incidence

21 days after inoculation with *Plasmodiophora,* statistics of the disease incidence and disease index were performed using a grading system based on *Plasmodiophora.* The grading criteria for *Arabidopsis thaliana* using the grading system based on *Plasmodiophora* are as follows: Grade 0, the roots of *Arabidopsis thaliana* are not infected and the structure of the root system is intact; Grade 1, there are very small galls on the lateral roots and the main root is not affected; Grade 2, there are galls on both lateral roots and main roots, but some root structures are intact and unaffected; Grade 3, both main roots and lateral roots are infected; Grade 4, the lateral roots and root hairs of *Arabidopsis thaliana* are completely damaged, leaving only an enlarged, club-shaped main root. The calculation formula of disease incidence = (number of diseased plants/total number of inoculated plants)×100%.

### 3. Analysis of relative biomass of Plasmodiophora

21 days after inoculation with *Plasmodiophora,* the underground parts of each material were collected, the total DNA was extracted from the underground parts using the CTAB method, and the expression levels of the *Arabidopsis thaliana* housekeeping gene *At-Actin* and the *Plasmodiophora* housekeeping gene *Pb-Actin* in different materials were detected using quantitative real-time PCR. The specific primer sequences are as follows:
Actin-RT-F: 5'-AACTCTCCCGCTATGTATGTCG-3' (sequence 6);
Actin-RT-R: 5'-AACCCTCGTAGATTGGCACA-3' (sequence 7);
Pb-Actin-RT-F : 5'-CACCGACTACCTGATGAA-3' (sequence 8);
Pb-Actin-RT-R: 5'-CAGCTTCTCCTTGATGT-3' (sequence 9).

The disease symptoms, disease incidence, disease index, and biomass of *Plasmodiophora* of plants 21 days after inoculation with *Plasmodiophora* are shown in FIGS. 1A-C. The results show that 21 days after inoculation, the root development of the clubroot-resistant material Est-1 is basically normal, with only a small number of plants showing mild symptoms; while the roots of the clubroot-susceptible material L*er* form spindle-shaped galls. Furthermore, the biomass of *Plasmodiophora* in the clubroot-susceptible material Ler is nearly 100 times that in the clubroot-resistant material.

The RCR1 genomic sequences and promoter regions of the clubroot-susceptible material Ler and the clubroot-resistant material Est-1 were obtained from the NCBI database. Alignment analysis using DNAMAN reveals that, compared with *RCR1*^{Est}, the *RCR1*^{L*er*} promoter region has a deletion of approximately 700 bp, and there is one amino acid mutation in the coding region of *RCR1,* i.e., a mutation of valine to isoleucine (FIG. 2D).

### 4. Detection of programmed cell death in tobacco

The ability of *RCR1*^{L*er*} and *RCR1*^{Est-1} to trigger programmed cell death was detected. The specific steps are as follows:
1) DNA was extracted from *Arabidopsis thaliana* ecotypes Est-1 and Ler, respectively. PCR amplification was performed using RCR1-per8-F and RCR1-per8-R to obtain the *RCR1* coding sequences *RCR1*^{Est} and *RCR1*^{L*er*} of the two ecotypes, respectively, with the nucleotide sequences as shown in sequence 1 and sequence 2, respectively. The pER8 vector was then double-digested with restriction enzymes Sall and Csp45l. Using in-Funion homologous recombination technology, the RCR1 coding sequences (*RCR1*^{Est} and *RCR1*^{L*er*}) were respectively ligated into the Sall and Csp45l restriction sites of the pER8 vector to obtain estrogen-inducible vectors pER8-*RCR1*^{Est} and pER8-*RCR1*^{L*er*}, respectively. Finally, the pER8-*RCR1*^{Est} and pER8-*RCR1*^{L*er*} were respectively introduced into *Agrobacterium* GV3101, and positive monoclonal clones were picked. The primer sequences are as follows:
   RCR1-per8-F: 5'-ACACGCTGAAGCTAGTCGACATGGAGACTGTCTCCGCCG-3' (sequence 10);
   RCR1-per8-R: 5'-TGGTCTTTGTAGTCTTCGAATTAAACTGGTGGTAACTGAG-3' (sequence 11).
2 ) Single colonies of *Agrobacterium* carrying and the pER8-*RCR1*^{Est} vector and the pER8-*RCR1*^{L*er*} vector were picked and cultured in LB medium overnight at 28°C and 220 rpm, respectively. The cultures were centrifuged at 4000 rpm for 10 minutes, and the bacterial cells were collected. The obtained bacterial cells were washed twice with water, and then diluted with sterile water to OD = 1.
3) The *Agrobacterium* (OD = 1) carrying either the pER8-*RCR1*^{Est-1} vector or the pER8-*RCR1*^{Ler} vector were injected into the abaxial side of *Nicotiana benthamiana,* respectively. After 24 hours, 4 µM estrogen was injected. After 48 hours, cell death was observed under ultraviolet light.

The results show that both *RCR1*^{Est} and *RCR1*^{L*er*} can trigger programmed cell death in tobacco.

### Example 2. The RCR1^{Est} promoter contains important elements responsive to Plasmodiophora infection

### (1) Strong induction of RCR1^{Est} expression in the underground parts of Arabidopsis thaliana by Plasmodiophora

According to the method in Example 1, the clubroot-susceptible material Ler and the clubroot-resistant material Est-1 were inoculated with *Plasmodiophora.* 11 days after inoculation, the underground parts of the clubroot-susceptible material Ler and the clubroot-resistant material Est-1 were collected, respectively. The expression level of *RCR1* in the clubroot-susceptible material L*er* and clubroot-resistant material Est-1 was detected using quantitative real-time PCR. The primer sequences are as follows:
Actin-RT-F: 5'-AACTCTCCCGCTATGTATGTCG-3' (sequence 6);
Actin-RT-R: 5'-AACCCTCGTAGATTGGCACA-3' (sequence 7);
RCR1-RT-F: 5'-GTGAACCGGTGAAGTTCACGAC-3' (sequence 12);
RCR1-RT-R: 5'-GAGGTGGACAGGCACTGGGT-3' (sequence 13).

The results show that *Plasmodiophora* can activate RCR1 expression in the underground parts of the clubroot-resistant material Est-1, but not in the clubroot-susceptible material Ler.(FIG. 2A).

### (2) Analysis of activities of RCR1^{Est} and RCR1^{Ler} promoters

To verify whether the difference in RCR1 expression between the clubroot-resistant material Est-1 and the clubroot-susceptible material Ler after inoculation with *Plasmodiophora* was due to the different RCR1 promoters in the two materials, the activities of the *RCR1*^{Est} and *RCR1*^{L*er*} promoters under conditions with or without inoculation with *Plasmodiophora* were further analyzed. The specific steps are as follows:
1. Obtaining *RCR1*^{Est} and *RCR1*^{L*er*} promoter fragments

DNA was extracted from the clubroot-susceptible material Ler and the clubroot-resistant material Est-1, respectively. PCR amplification was performed using primers RCR1-PF and RCR1-PR to obtain PCR products with a size of 3727 bp and 3036 bp, respectively, with the nucleotide sequences as shown in sequence 3 and sequence 4, respectively (designated as *pRCR1*^{Est} and *pRCR1*^{L*er*}, respectively). The primer sequences are as follows:
RCR1-PF: 5'-CGACGGCCAGTGCCAAGCTTAGCCACACAAACTCCCATCT-3' (sequence 14);
RCR1-PR: 5'-GCGCGCCTCGAGATCCTCTAGAAATTTGTATCTTTGCAGTTTT-3' (sequence 15).

### 2. Obtaining RCR1 promoter-driven GUS plant binary expression vector

The pCAMBIA1300-GUS vector was double-digested with restriction enzymes Hindlll and Xbal and recovered to obtain a pCAMBIA1300 vector backbone. Then, using in-Funion homologous recombination technology, the pCAMBIA1300 vector backbone was reacted with the PCR products obtained in step 1, respectively, to obtain recombinant expression vectors *pRCR1*^{Est}*::GUS* and *pRCR1*^{L}*^{er}::GUS*, respectively, which were sequenced.

The sequencing results show that the recombinant expression vector *pRCR1*^{Est}*::GUS* is a vector obtained by ligating the DNA molecule as shown in sequence 3 into the Hindlll and Xbal restriction sites of the pCAMBIA1300-GUS vector,
and that the recombinant expression vector *pRCR1*^{L}*^{er}::GUS* is a vector obtained by ligating the DNA molecule as shown in sequence 4 into the Hindlll and Xbal restriction sites of the pCAMBIA1300-GUS vector.

### 3. Obtaining transgenic Arabidopsis thaliana

### 1) Obtaining of Agrobacterium carrying either pRCR1^{Est}::GUS or pRCR1^{L}^{er}::GUS

The recombinant expression vectors *pRCR1*^{Est}*::GUS* and *pRCR1*^{L}*^{er}::GUS* obtained in step 2 were introduced into *Agrobacterium* GV3101 by electroporation, respectively. The transformed strains were then spread on LB solid culture dishes containing kanamycin and gentamicin, and cultured at 28°C for 36 hours to screen positive monoclonal strains.

### 2) Transformation

Using the *Agrobacterium tumefaciens-mediated Arabidopsis thaliana* transformation method, the *Agrobacterium* carrying *pRCR1*^{Est}*::GUS* and the *Agrobacterium* carrying *pRCR1*^{L}*^{er}::GUS* obtained in step 1) were transformed into *Arabidopsis thaliana* ecotype Col-0, respectively. The specific steps are as follows:
I. Preparation of *Arabidopsis thaliana* transformation recipient: *Arabidopsis thaliana* at the flowering stage was selected, the already opened flowers were removed, and the remaining *Arabidopsis thaliana* flower buds were used as the recipient for *Agrobacterium* infection.
II. Culture of *Agrobacterium:* A small amount of *Agrobacterium* stock was taken and streaked on LB solid medium (containing 50 mg/L kanamycin and 50 mg/L gentamicin), and cultured at 28°C in the dark for 12 hours. A few bacterial cells were picked and inoculated into 2 mL of LB liquid medium (containing the corresponding antibiotics) and cultured at 28°C for 12 hours. Then 2 mL of the LB liquid culture was transferred to 300 mL of LB liquid medium (containing the corresponding antibiotics) and cultured for about 10 hours. The culture was centrifuged at 4000 rpm to collect the bacterial cells. The bacterial cells were diluted with 5% sucrose solution to an OD of about 0.9, and a surfactant (silwet L-77) was added at a ratio of 0.17% for transformation.
III. Infection of *Arabidopsis thaliana* flower buds with *Agrobacterium:* The diluted bacterial suspension was placed in a cylindrical glass bottle. The *Arabidopsis thaliana* containing only flower buds was inverted into the bacterial suspension for 4-5 minutes. Then the plants were placed horizontally. After 24 hours in the dark, the plants grew vertically.
IV. Screening of resistant plants: The seeds harvested from the transgenic plants were evenly spread on a 1/2 MS medium containing 25 mg/L hygromycin B and 50 mg/L carbenicillin for screening. After about 10 days, normally growing seedlings were selected and transferred to the greenhouse for cultivation.
V. Identification of positive plants: After the plants grew in the culture chamber for 10 days, 1-2 leaves were selected, the total DNA was extracted using the CTAB method, and DNA fragments in the genome were amplified using primer hyg-F and primer hyg-R. The primer sequences for determining positive plants are as follows:
   hyg-F: AAGCCTGAACTCACCGCGA (sequence 16);
   hyg-R: GTTTCCACTATCGGCGAGTAC (sequence 17).

### 4. Analyzing promoter activity

According to the method in Example 1, T2 generation transgenic plants carrying either *pRCR1*^{Est}*::GUS* or *pRCR1*^{L}*^{er}::GUS* were respectively inoculated with *Plasmodiophora.* After 11 days, the underground parts were harvested respectively, and the GUS activity in different plant materials was detected using the 4-MUG fluorometric assay with a *GUS* Gene quantitative detection kit (Coolaber). Meanwhile, the plants in the group without inoculation with *Plasmodiophora* were used as a control group (the control group was inoculated with an equivalent amount of water instead of the *Plasmodiophora* suspension used in the experimental group).

The results show that compared with the control group without inoculation with *Plasmodiophora, Plasmodiophora* infection can significantly increase the GUS activity in the transgenic material *pRCR1*^{Est}*::GUS*, while the GUS activity in the transgenic material *pRCR1*^{L}*^{er}::GUS* is not affected by *Plasmodiophora* (FIG. 2B). This indicates that the *pRCR1*^{Est} promoter contains elements responsive to *Plasmodiophora.*

### (3) Obtaining transgenic material pRCR1^{Est}::nls-3*mVenus and performing fluorescence observation

To verify whether the *pRCR1*^{Est} promoter was a Plasmodiophora-responsive promoter, the present invention further constructed an expression vector of a green fluorescent protein mVENUS with a nuclear localization signal driven by the *pRCR1*^{Est} promoter, and observed its expression pattern. The specific steps are as follows:
1. Obtaining transgenic material *pRCR1*^{Est}*::nls-3***mVenus* and performing fluorescence observation
   1) DNA was extracted from *Arabidopsis thaliana* ecotype Est-1. PCR amplification was performed using primers RCR1-VF and RCR1-VR to obtain the *pRCR1*^{Est} promoter with a size of 3727 bp, with the nucleotide sequence as shown in sequence 3. The primer sequences are as follows:
      RCR1-VF: 5'-TCGAGGTCGACGGTATCGATAAGCTTAGCCAGACAAACTCCCATCT -3' (sequence 18);
      RCR1-VR: 5'-TTCCTCTTCTTCTTTGGCATGAATTCAATTTGTATCTTTGCAGTT -3' (sequence 19).
   2). The pGREEN-nls-3*mVenus vector was double-digested with restriction enzymes Hindlll and EcoRI and recovered to obtain a pGREEN-nls-3*mVenus vector backbone. Then, using in-Funion homologous recombination technology, the pGREEN-nls-3*mVenus vector backbone was reacted with the PCR products (*pRCR1*^{Est} promoter with a size of 3727 bp) obtained in step 1) to obtain a recombinant expression vector *pRCR1*^{Est}*::nls-3***mVenus*, which was sequenced.

The sequencing results show that the recombinant expression vector *pRCR1*^{Est}*::nls-3***mVenus* is a vector obtained by ligating the DNA molecule as shown in sequence 3 into the Hindlll and EcoRI restriction sites of the pGREEN-nls-3*mVenus vector.

3) The transgenic plants were prepared according to the method in step 2.

### 2. Fluorescence observation of the transgenic plant pRCR1^{Est}::nls-3*mVenus

According to the method in Example 1, T2 generation transgenic plants carrying *pRCR1*^{Est}*::nls-3*mVenus* were inoculated with *Plasmodiophora.* After 11 days, the underground parts were harvested, respectively, stained with 50 µg/mL propidium iodide (PI) for 10 minutes, then rinsed with water for 5 minutes, and observed and photographed using a confocal fluorescence microscope (TIRF3 & Axioimager.Z2, ZEISS). Meanwhile, the plants in the group without inoculation with *Plasmodiophora* were used as a control group (the control group was inoculated with an equivalent amount of water instead of the *Plasmodiophora* suspension used in the experimental group).

The results show that compared with the control group without inoculation with *Plasmodiophora, Plasmodiophora* infection can significantly induce the expression of the green fluorescent protein mVENUS (FIG. 2C). This further indicates that the *RCR1*^{Est} promoter contains *Plasmodiophora-*responsive elements.

As can be seen from the combination of Example 1 and Example 2, the clubroot-susceptible material Ler contains a functional RCR1^{L*er*} protein. However, due to a deletion mutation in its promoter, which results in the loss of Plasmodiophora-responsive elements, *Plasmodiophora* infection cannot activate high expression of *RCR1*^{L*er*} in the clubroot-susceptible material Ler, thereby causing the clubroot-susceptible material L*er* to exhibit a phenotype that is highly sensitive to *Plasmodiophora.* This indicates that the *pRCR1*^{Est} promoter is important for mediating resistance to *Plasmodiophora.*

### Example 3. The RCR1^{Est} promoter is essential for RCR1-mediated resistance to Plasmodiophora

### I. Obtaining of transgenic material

1. Using the DNA of the clubroot-resistant material Est-1 as a template, PCR amplification was performed using primers RCR1-EF and RCR1-ER to obtain the *pRCR1*^{Est} promoter with a size of 3727 bp. Then, using the DNA of the clubroot-susceptible material Ler as a template, PCR amplification was performed using primers RCR1^{L*er*}-F and RCR1^{L*er*}-R to obtain the *RCR*^{L*er*} coding sequence and UTR region with a size of 5147 bp. Finally, the PCR product *pRCR1*^{Est}-*RCR*^{L*er*} was obtained by overlap PCR, with the nucleotide sequence as shown in sequence 5. The sequences of the primers used are as follows:
   RCR1-EF: 5'-GGCCCCCCCTCGAGGTCGACAGCCACACAAACTCCCATCT-3' (sequence 20);
   RCR1-ER: 5'-ACGGCGGAGACAGTCTCCATAATTTGTATCTTTGCAGTTT-3' (sequence 21);
   RCR1^{L*er*}-F: 5'-GGCCCCCCCTCGAGGTCGACATGGAGACTGTCTCCGCCGT-3' (sequence 22);
   RCR1^{L*er*}-R: 5'-CATTAAAGCAGGACTCTAGAAGGAAACTACGTGTTTTAAA-3' (sequence 23).
2. The pGREEN-nls-3*mVenus vector was double-digested with restriction enzymes Sall and Xball and recovered to obtain a pGREEN vector backbone. Then, using in-Funion homologous recombination technology, the pGREEN vector backbone was reacted with the PCR product *pRCR1*^{Est}-*RCR*^{L*er*} obtained in step 1 to obtain a recombinant expression vector *pRCR1*^{Est}*::RCR*^{L*er*}, which was sequenced.

The sequencing results show that the recombinant expression vector *pRCR1*^{Est}*::RCR*^{L*er*} is a vector obtained by ligating the DNA molecule as shown in sequence 5 into the Sall and Xball restriction sites of the pGREEN vector.

3. The transgenic plant was obtained according to the method in Example 2.

### II. Analysis of resistance to Plasmodiophora in pRCR1^{Est}::RCR^{Ler} material

According to the method in Example 1, the T2 generation transgenic plants carrying *pRCR1*^{Est}*::RCR*^{L*er*} (obtained in step 1) were inoculated with *Plasmodiophora.* 21 days after inoculation, the disease symptoms of the transgenic plants carrying *pRCR1*^{Est}*::RCR*^{L*er*} were observed, the statistics of disease incidence and disease index were performed, and the biomass of *Plasmodiophora* was detected.

The results are shown in FIG. 3. The results show that 21 days after inoculation, the transgenic plant carrying *pRCR1*^{Est}*::RCR*^{L*er*} exhibits high resistance to *Plasmodiophora,* indicating that the *RCR1*^{Est} promoter is essential for the RCR1-mediated resistance to *Plasmodiophora.*

### Industrial applications

The present invention provides a DNA molecule, wherein the DNA molecule comprises important elements responsive to *Plasmodiophora* infection, and is a Plasmodiophora-responsive promoter. The DNA molecule is very important for mediating resistance to *Plasmodiophora,* and has an important application value.

## Claims

1. A DNA molecule, which is A1) or A2) as follows:
A1) a DNA molecule as shown in sequence 3;
A2) a DNA molecule having 50% or more identity to the nucleotide sequence as defined in A1) and having promoter function.

2. A biological material related to the DNA molecule as claimed in claim 1, wherein the biological material is any one of B1) to B4) as follows:
B1) an expression cassette containing the DNA molecule as claimed in claim 1;
B2) a recombinant vector containing the DNA molecule as claimed in claim 1, or a recombinant vector containing the expression cassette of B1);
B3) a recombinant microorganism containing the DNA molecule as claimed in claim 1, a recombinant microorganism containing the expression cassette of B1), or a recombinant microorganism containing the recombinant vector of B2);
B4) a transgenic plant cell line containing the DNA molecule as claimed in claim 1, or a transgenic plant cell line containing the expression cassette of B1).

3. The use of the DNA molecule as claimed in claim 1 as a promoter.

4. The use of the DNA molecule as claimed in claim 1 as a Plasmodiophora-responsive promoter.

5. The use of the DNA molecule as claimed in claim 1 in initiating the expression of a gene of interest.

6. The use of the DNA molecule as claimed in claim 1 in increasing the expression level of a gene of interest.

7. The use of the biological material as claimed in claim 2 in initiating the expression of a gene of interest.

8. The use of the biological material as claimed in claim 2 in increasing the expression level of a gene of interest.

9. The use of the DNA molecule as claimed in claim 1 or the biological material as claimed in claim 2 in the preparation or breeding of a transgenic plant.

10. The use of the DNA molecule as claimed in claim 1 or the biological material as claimed in claim 2 in plant breeding.

11. The use as claimed in claim 9 or 10, wherein the plant is a dicotyledonous plant or a monocotyledonous plant.

12. The use as claimed in claim 11, wherein the dicotyledonous plant is a cruciferous plant.

13. The use as claimed in claim 12, wherein the cruciferous plant is *Arabidopsis thaliana* or *Brassica napus.*

14. A method for expressing a gene of interest or a method for increasing the expression level of a gene of interest, wherein the method is any one of C1)-C4) as follows:
C1) comprising the step of using the DNA molecule as claimed in claim 1 as a promoter or a Plasmodiophora-responsive promoter to initiate the expression of the gene of interest;
C2) comprising the step of inserting the DNA molecule as claimed in claim 1 upstream of the gene of interest or an enhancer to initiate the expression of the gene of interest;
C3) comprising the step of inserting the gene of interest downstream of the DNA molecule in the expression cassette as claimed in claim 2 to initiate the expression of the gene of interest by the DNA molecule;
C4) comprising the step of inserting the gene of interest downstream of the DNA molecule in the recombinant vector as claimed in claim 2 to initiate expression of the gene of interest by the DNA molecule.

15. The use of the method as claimed in claim 14 in the preparation or breeding of a transgenic plant.
